**Europäisches Patentamt**

**European Patent Office**

**Office européen des brevets**

(19)

(11) Veröffentlichungsnummer: **0 256 546 B1**

(12) **EUROPÄISCHE PATENTSCHRIFT**

(45) Veröffentlichungstag der Patentschrift: **07.08.91**

(51) Int. Cl.⁵: **A61M 25/00**

(21) Anmeldenummer: **87111903.8**

(22) Anmeldetag: **17.08.87**

(54) **Vorrichtung zum Plazieren eines Ernährungs-Schlauches am Magen des menschlichen oder tierischen Körpers.**

(30) Priorität: **18.08.86 DE 3628006**

(43) Veröffentlichungstag der Anmeldung:
**24.02.88 Patentblatt 88/08**

(45) Bekanntmachung des Hinweises auf die
Patenterteilung:
**07.08.91 Patentblatt 91/32**

(84) Benannte Vertragsstaaten:
**AT BE CH DE ES FR GB GR IT LI LU NL SE**

(56) Entgegenhaltungen:
**GB-A- 2 032 278**
**US-A- 3 961 632**
**US-A- 4 134 405**

(73) Patentinhaber: **Frimberger, Erintrud**
**Josef-Kösel-Weg 10**
**W-8960 Kempten(DE)**

(72) Erfinder: **Frimberger Eckart, Dr.**
**Tristanstrasse 12**
**W-8000 München 40(DE)**

(74) Vertreter: **Mitscherlich, Hans, Dipl.-Ing. et al**
**Patentanwälte Dipl.-Ing. H. Mitscherlich**
**Dipl.-Ing. K. Gunschmann Dipl.-Ing.**
**Dr.rer.nat. W. Körber Dipl.-Ing. J. Schmidt-**
**Evers Dipl.-Ing. W. Melzer Steinsdorfstrasse**
**10**
**W-8000 München 22(DE)**

## Beschreibung

Die Erfindung bezieht sich auf eine Vorrichtung nach dem Oberbegriff des Anspruches 1.

Bei Patienten, bei denen eine Ernährung durch die Speiseröhre, beispielsweise in Folge einer Schluckstörung nach Schlaganfall oder bei einem Speiseröhrentumor nicht möglich ist, kann die Ernährung über eine sognannte Fistel erfolgen, die von der Bauchoberfläche direkt in den Magen führt. Früher wurden solche Fisteln vom Chirurgen operativ angelegt (Witzelfistel); heutzutage können sie in Form von Ernährungs-Schläuchen einfacher endoskopisch plaziert werden. Dabei wird wie folgt vorgegangen:

Ein flexibles Gastroskop wird in den Magen eingeführt, und unter endoskopischer Kontrolle wird von außen eine Hohlnadel in den Magen eingestochen, auf der eine Hülse aus Kunststoff angeordnet ist. Die Hohlnadel wird danach aus der Hülse herausgezogen, während die Hülse im Einstich verbleibt. Der nächste Schritt besteht darin, einen Faden durch die Hülse in den Magen einzuführen, der dann von innen mittels eines über den endoskopischen Instrumentierkanal eingeführten Greifwerkzeugs erfaßt und nach extrakorporal gezogen wird, wobei es erforderlich ist, auch das Gastroskop herauszuziehen, um das freie Ende des Fadens aus dem Instrumentierkanal herausziehen zu können. Dieses extrakorporale Ende 1 des Fadens wird dann außerhalb des Körpers des Patienten an der Spitze des Ernährungs-Schlauches befestigt. Durch Zug am fistelseitigen Ende des Fadens wird der Schlauch dann über den Mund und die Speiseröhre zunächst in den Magen und dann in den Einstich eingezogen, wobei die Hülse gleichzeitig aus der Magenwand und der Bauchdecke herausgezogen oder herausgestossen wird. Die Endstellung des Schlauches wird durch ein ringförmiges Begrenzungsteil an seinem Umfang dadurch definiert, daß das Begrenzungsteil an die Innenwand des Magens gelangt und somit den Durchzug des Schlauches verhindert. Nach dem Lösen des Fadens vom nun herausragenden Ende des Schlauches und Zuschneiden dieses Endes auf eine erwünschte Länge, kann die Ernährung durch den Schlauch erfolgen. Die Fixierung dieses Schlauchendes ist durch ein Pflaster möglich.

Diese bekannte Vorrichtung erfordert für eine Plazierung des Schlauches einen beträchtlichen Behandlungs- und Zeitaufwand. Dies ist dadurch bedingt, daß die Verbindung zwischen dem Faden und dem Schlauch nur außerhalb des Körpers geschaffen werden kann, wozu der vorbeschriebene Behandlungsschritt, nämlich das Herausziehen des Fadens mittels des Gastrokops, erforderlich ist.

Der Erfindung liegt die Aufgabe zugrunde, eine eingangs bezeichnete Vorrichtung so weiterzubilden, daß die Verbindung zwischen dem fadenförmigen Zugelement und dem Schlauch innerhalb des Magens geschaffen werden kann. Diese Aufgabe wird durch die kennzeichnenden Merkmale des Anspruches 1 gelöst.

Bei der erfindungsgemäßen Ausgestaltung sind dem Schlauch und der Hohlnadel zugeordnete erste und zweite fadenförmige Zugglieder mit Kupplungsgliedern vorhanden, die unter endoskopischer Kontrolle leicht und einfach im Magen miteinander verbunden werden können. Dabei wird das der Hohlnadel zugeordnete Kupplungsglied durch eine spreizbare Schlaufe gebildet, wodurch sich die Verbindung nicht nur leicht verwirklichen läßt, sondern wodurch es auch in einfacher Weise möglich ist, dieses Zugglied durch die Hülse oder vorzugsweise die Hohlnadel einzuführen. Für das Kupplungsglied des ersten Zuggliedes sind mehrere Ausgestaltungen möglich, z.B. in Form eines Hakens einer kopfartigen Verdickung. Wesentlich ist, daß dieses Kupplungsglied durch die Schlaufe ergriffen werden kann. Aufgrund des verringerten Behandlungsaufwandes ergibt sich bei Benutzung der erfindungsgemäßen Vorrichtung sowohl für die behandelnde Person als auch für den Patienten eine bedeutend angenehmere Behandlung.

Gemäß den Ansprüchen 2 und 3 wird auf an sich bekannte und bewährte Maßnahmen zur Schaffung einer sich selbsttätig öffenden Schlaufe zurückgegriffen.

Die im Anspruch 4 enthaltene Ausgestaltung zeichnet sich durch besondere Einfachheit aus, weil das schlauchseitige Zugglied durch einen einfachen Drahtabschnitt gebildet ist. Dieser einfache Drahtabschnitt bildet sich automatisch zu einem wirksamen Kupplungsglied, wenn er durch die Schlaufe hindurchgesteckt wird, und anschließend die Schlaufe aus der Hohlnadel herausgezogen wird. Dabei stößt der Drahtabschnitt entweder gegen die Spitze der Hohlnadel oder gegen die Hülse und wird Dabei umgebogen, wobei sich ein wirksames Kupplungselement automatisch ausbildet.

Die Ausgestaltung nach Anspruch 5 ermöglicht es, den Schlauch und sein Zugglied außerhalb des Gastrokops anzuordnen. Zur Anordnung im bzw. Einführung durch das Gastroskop eignen sich z.Zt. gebräuchliche Gastroskope nicht.

Die Ausbildung nach Anspruch 6 ermöglicht es, trotz Verlaufs des Drahtabschnitts am Umfang des Gastroskops das freie Ende des Drahtabschnitts durch ein Instrument des Gastroskops längs zu verschieben. Es ist auch möglich, diese Längsverschiebung durch Zug von außen am Zugglied zu erreichen.

Die Ausgestaltung nach Anspruch 7 ist aus baulichen und hygienischen Gründen von wesentlicher Bedeutung. Es ist möglich, die sterile Verpakkung vor ihrer Durchführung durch den Mundraum als auch erst im Hohlraum des Magens zu öffnen. Im letzten Falle kann das Verpackungsmaterial nach dem Plazieren des Schlauchs mittels des Gastroskops extrakorporal entfernt werden.

Es ist im Rahmen der Erfindung möglich, die Hohlnadel und die durch sie in den Hohlraum des Magens einführbare Schlaufe in Form einer vorfertigbaren Baueinheit anzuordnen, die vorzugsweise steril verpackt sein kann. Es ist von Vorteil, die Schlaufe so anzuordnen, daß sie sich in ihrer Bereitsschaftsstellung, d.h. innerhalb der Hohlnadel, befindet.

Durch eine Abdeckkappe gemäß Anspruch 9 läßt sich die Öffnung von außen wirksam verschließen und schützen.

Dabei kann der Schlauch durch die Abdeckkappe herausgeführt sein, so daß es bei der Ernährung durch den Schlauch einer Entfernung der Abdeckkappe nicht bedarf.

Die Abdeckkappe läßt sich gemäß Anspruch 11 in einfacher und vorteilhafter Weise durch eine zwischen dem Schlauch und der Abdeckkappe wirksame Klemmvorrichtung befestigen, wodurch der Schlauch insgesamt eine Fixierung erhält, weil sein ringförmiges Begrenzungsteil an der Innenwand des Magens anliegt.

Die Ausbildungen nach den Ansprüche 12 bis 14 dienen dazu, das herausragende Ende des Schlauches in der Abdeckkappe zu versenken, wodurch Störungen durch das herausragende Ende vermieden sind.

Gemäß Anspruch 15 ist an dem Schlauch ein Flansch angeordnet, der bei im Patienten plaziertem Schlauch an der Innenseite der Magenwand anliegt, um ein Herausrutschen des Ernährungsschlauches aus der Magenwand während der Ernährungsmanipulation zu vermeiden. Wird der Patient so ernährt, daß die Nahrung in den Magen gepumpt wird, endet der Ernährungsschlauch am inneren magenseitigen Ende des Flansches oder der Schlauch ragt mit einem inneren Schlauchteil einige Zentimeter von dem Flansch entfernt in den Magen hinein.

Bei Kranken mit gestörter Schluckfunktion, z.B. nach einem Schlaganfall, darf die Nahrung nicht in den Magen geleitet werden, da sie infolge der Magenkontraktionen in die Speiseröhre und von da ins Bronchialsystem zurückfließen kann mit dem Risiko einer Aspirationspneumonie. Zur Vermeidung dieser Komplikation ist man bestrebt, das innere Schlauchteil nicht im Magen enden zu lassen, sondern in den Dünndarm zu plazieren, je weiter, desto besser, da sich mit der Distanz das Risiko des Rückflusses von Nahrung aus dem Dünndarm Richtung Magen, Speiseröhre und Lunge vermindert. Um diese Plazierung eines etwa 20 bis 30 cm langen inneren Schlauchteiles im Dünndarm zu ermöglichen, bildet nach Anspruch 16 der innere, orale Schlauchteil eine Schlaufe und ist mit seinem hinteren Ende lösbar an dem Flansch befestigt.

Nachfolgend wird die Erfindung anhand eines in vereinfachten Zeichnungen dargestellten bevorzugten Ausführungsbeispiels erläutert. Es zeigt:

Fig. 1     eine erfindungsgemäß ausgestaltete Vorrichtung zum Plazieren eines Ernährungs-Schlauches am Magen eines menschlichen Körpers während der Plazierung;

Fig. 2     die in Fig. 1 mit X gekennzeichnete Einzelheit in vergrößerter Darstellung;

Fig. 3     bis 6 der Fig. 2 entsprechende Ansichten der Vorrichtung während unterschiedlicher Behandlungsstadien;

Fig. 7     eine Ansicht entsprechend Fig. 5 mit innerem Schlauchteil als Schlaufe;

Fig. 8     eine vergrößerte Ansicht des Bereiches A in Fig. 7.

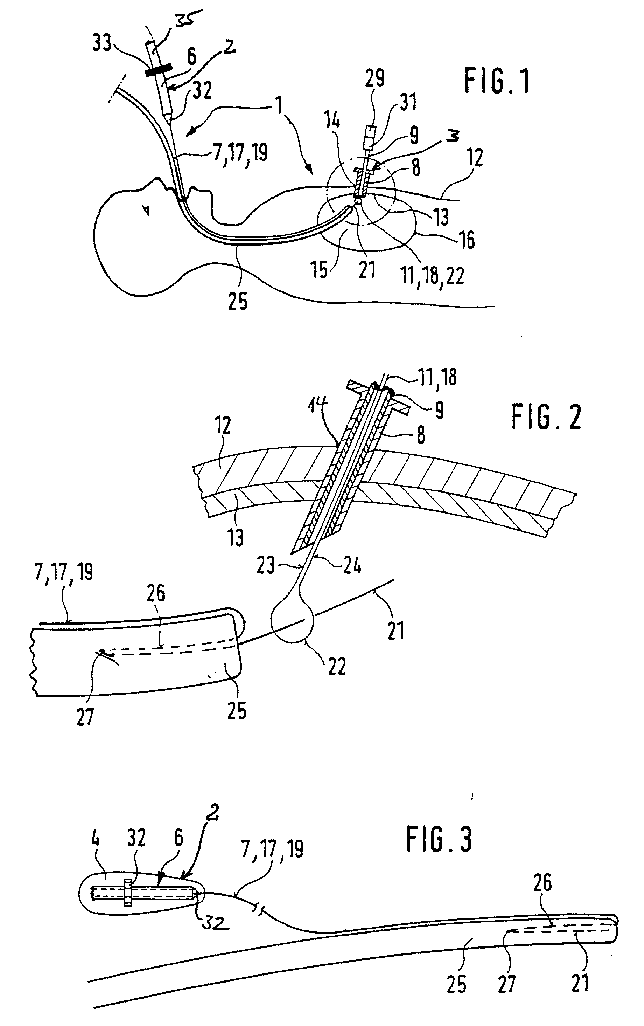

Wie aus Fig. 1 zu entnehmen ist, besteht die allgemein mit 1 bezeichnete Vorrichtung aus zwei steril verpackten Vorrichtungsteilen 2,3, von denen die Verpackung des Vorrichtungsteils 2 mit 4 bezeichnet ist, und von denen der mit 2 bezeichnete Vorrichtungsteil den Ernährungs-Schlauch 6 sowie ein allgemein mit 7 bezeichnetes Verbindungsteil und der mit 3 bezeichnete Vorrichtungsteil eine von einer Hülse 8 umgebene Hohlnadel 9 mit einem zweiten Verbindungsteil 11 aufweist.

Die Vorrichtung 1 dient dazu, in der Bauchdecke 12 und in der ihr zugewandten Magenwand 13 eine Öffnung 14 anzubringen und den Schlauch 6 von innen, d.h. durch den Mundraum, die Speiseröhre und den Hohlraum 15 des Magens 16 hindurch in die Öffnung 14 einzusetzen.

Beide vorbeschriebenen Verbindungsteile 7,11 sind faden-bzw. drahtförmige Zugglieder 17,18, von denen das dem Schlauch 6 zugeordnete Zugglied 17 durch einen biegsamen, sich in seiner Ausgangsposition gerade erstreckenden Draht 19 gebildet ist, der länger bemessen ist, als der Abstand zwischen dem Hohlraum 15 des Magens 16 und dem Mund des Patienten, so daß der Schlauch 6 sich außerhalb des Körpers befindet, wenn das mit 21 bezeichnete innere Ende des Zuggliedes 17 sich in seiner zur Kupplung bzw. Verbindung bereiten Position befindet. Das der Hohlnadel 9 zugeordnete Zugglied 18 ist eine Schlaufe 22 aus Draht, die aus einer in der Hohlnadel 9 eingezogenen Position in den Hohlraum 15 des Magens 16 schiebbar ist und sich dabei automatisch öffnet. Letzteres kann dadurch bewerkstelligt werden, daß nur einer der an der Schlaufenspitze miteinander verbundenen Schenkel 23,24 der Schlaufe 22 verschoben wird. Es ist auch möglich, die beiden Schenkel 23,24 mit einer Spannung zu versehen, die sie voneinander wegbiegt, wodurch ebenfalls eine selbsttätige Öffnung der Schlaufe 22 erreicht wird, wenn diese aus der Hohlnadel 9 herausgeschoben wird.

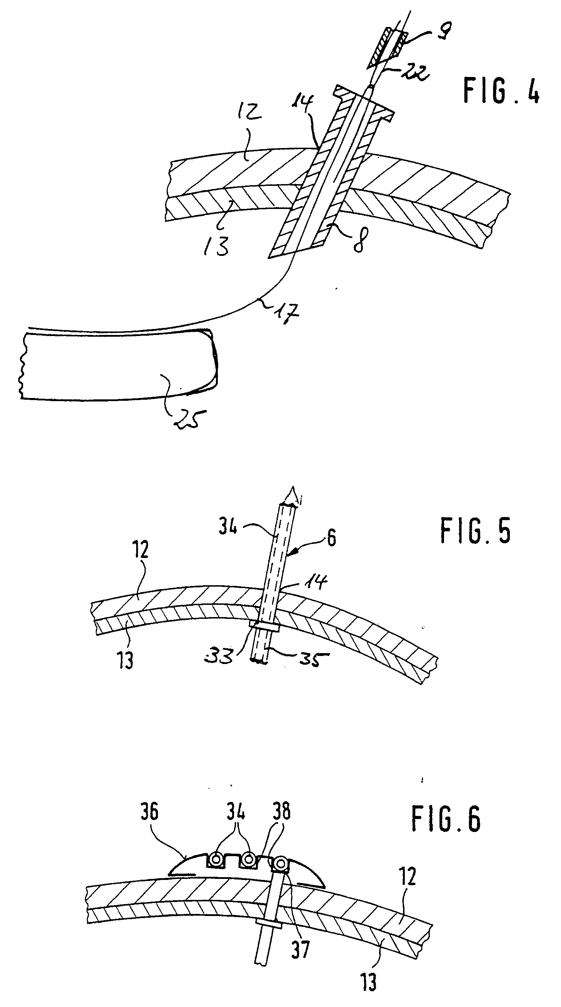

Zur Plazierung des Schlauches 6 wird zunächst das Einführungselement 25 eines im einzelnen nicht dargestellten Gastroskops in den Hohlraum 15 des Magens 16 durch den Mund des Patienten eingeführt. Mit dem Einführungselement 25 wird auch das Verbindungsteil 7 bzw. das Zugglied 17 des Schlauches 6 eingführt, das sich in Längsrichtung am Umfang des Einführungselementes 25 erstreckt. Das Zugglied 17 ist am inneren Ende des Einführungselementes 25 Z-förmig gebogen, d.h es erstreckt sich mit einem mit 26 bezeichneten eingebogenen Abschnitt in den nicht dargestellten Instrumentierkanal des Einführungselemtes 25 hinein und dann wieder von einer mit 27 bezeichneten Faltstelle aus in Richtung auf den Magen 16, wobei das innere Ende 21 des Zuggliedes 17 mittels eines im Instrumentenkanal verschiebbaren Schubgliedes (nicht dargestellt) aus einer in den Instrumentenkanal eingetauchten Position in eine aus dem Instrumentenkanal herausragenden Position verschoben werden kann. Fig. 2 zeigt das innere Ende 21 des Zuggliedes 17 in der herausgeschobenen Position, in der das innere Ende 21 zwecks Verbindung mit der Schlaufe 22 durch letztere hindurchgesteckt ist. Wenn aus dieser Position heraus die Schlaufe 22 allein oder auch gegebenenfalls mit der Hohlnadel 9 jedoch bei in der Öffnung 14 verbleibender Hülse 8 zurückgezogen wird, dann wird das innere Ende 21 des Zuggliedes 17 aufgrund des Anschlages gegen die Hohlnadel 9 oder die Hülse 8 hakenförmig verbogen, wodurch eine sicher funktionierende Ankoppelung geschaffen wird (Fig. 4). Durch weiteren Zug an der Schlaufe 22 bzw. an der Hohlnadel 9, deren Griffe vereinfacht dargestellt und mit 29,31 bezeichnet sind, wird nach vorheriger Entfernung des Einführungselementes 25 der Schlauch 6 durch den Mund, die Speiseröhre und den Hohlraum 15 des Magens 16 hindurch in die Öffnung 14 hineingezogen, wobei das Hineinziehen aufgrund eines Konus 32 an der Spitze des Schlauches 6 erleichtert wird. In der in der Öffnung 14 eingezogenen Position nimmt der Schlauch 6 die in Fig. 5 dargestellte Stellung ein, in der ein umfangsseitig vorspringender Flansch 33 innenseitig an der Magenwand 13 anliegt (Fig. 5). Die Hülse 8 wird beim Einziehen des Schlauches 6 in die Öffnung 14 herausgeschoben. Nunmehr bedarf es gegebenenfalls einer Abschneidung des von der Bauchdecke 12 frei abstehenden Endes 34 des Schlauches 6, um letzteren auf eine gewünschte Länge zu bringen. Nunmehr kann die Ernährung durch den Schlauch 6 erfolgen, wobei nicht dargestellte Zuführungsteile in bekannter Weise an das Ende 34 des Schlauches 6 anzubringen sind.

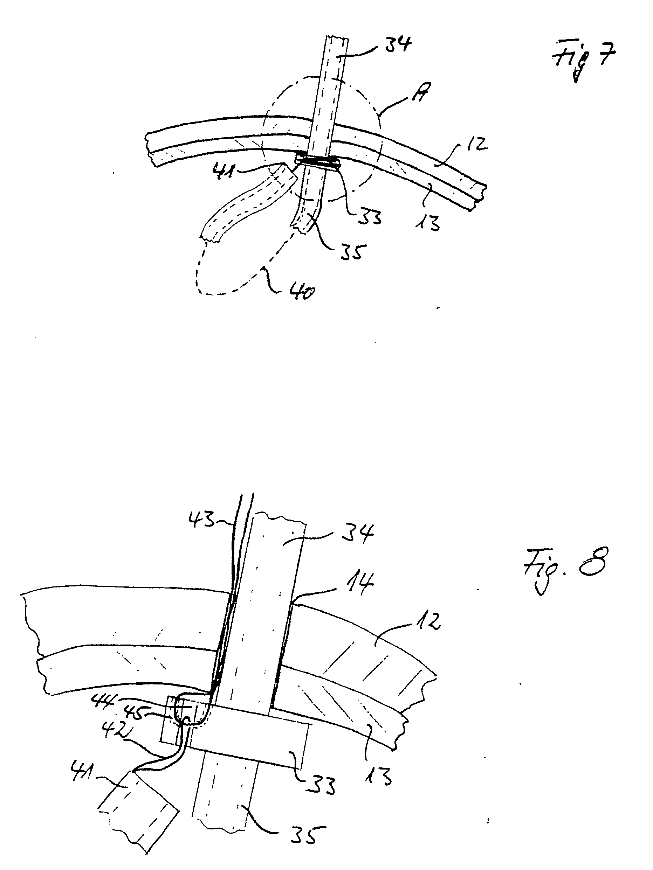

Die Ausbildung des Ernährungsschlauches 6 nach Fig. 7 und 8 erleichtert das eingangs in Verbindung mit Anspruch 16 erläuterte Plazieren des inneren Schlauchteils 35 in den nicht dargestellten, sich an den Magen 16 anschließenden Dünndarmbereich des Patienten. Bei der in Verbindung mit Fig. 1 bis 6 beschriebenen Plazierung des Ernährungsschlauches 6 kommt das innere Schlauchteil 35 in den Magen 16 zu liegen. Benötigt man für die Plazierung des inneren Schlauchteils 35 in den Dünndarm eine größere Länge des Schlauchteiles beispielsweise 20 bis 30 cm, so kommt dieser verlängerte innere Schlauchteil 35 nach der Plazierung in der Speiseröhre des Patienten zu liegen. Dort müßte dieses Ende mit dem Endoskop gefaßt und in den Dünndarm transportiert werden. Dazu muß das Endoskop extrahiert werden, das innere Schlauchende erfaßt und durch erneutes Einführen des Endoskops in den Dünndarm gebracht werden. Um das Extrahieren und erneute Einführen des Endoskopes zu vermeiden, ist gemäß Fig. 7 das innere Schlauchteil 35 als Schlaufe 40 ausgebildet und mit seinem inneren Ende 41 durch geeignete, beispielsweise in Fig. 8 dargestellte Mittel lösbar an dem Flansch 33 befestigt. Diese Mittel können beispielsweise aus je einer am Ende 41 und an dem Flansch 33 angeordneten Fadenschlaufe 42 und 43 gebildet sein. Die Fadenschlaufe 52 am Schlauchende 41 ragt in einen Schlitz 44 im Flansch 33 und wird von der Schlaufe 43, die durch Öffnungen 45 quer zum Schlitz 44 verläuft, gehalten. Die Schlaufe 43 ist mit ihren Enden im Bereich des Konus 32 oder am Zugglied 17 befestigt. Die Schlaufe 43 wird mit dem beschriebenen Ablängen des äußeren Schlauchteiles 34 abgeschnitten, und der die Schlaufe 43 bildende Faden kann herausgezogen werden und gibt die Schlaufe 42 und damit das innere Ende 41 des Schlauchteiles 35 frei, so daß dieses Ende mit Hilfe des Endoskops erfaßt und in den Dünndarm plaziert werden kann.

Es ist vorteilhaft, die Öffnung 14 von außen durch eine Abdeckkappe 36 zu verschließen, die ein Loch 37 aufweist, durch das hindurch das Ende 34 des Schlauches 6 hindurchgeführt ist. Der Querschnitt des Loches 37 kann geringfügig kleiner bemessen sein, als der Querschnitt des Schlauches 6, so daß die Abdeckkappe 36 mit einer gewissen Klemmwirkung auf dem Schlauch 6 sitzt, wodurch sowohl eine Abdichtung am Rand des Loches 37 als auch eine Halterung erzielt wird, mit der die Abdeckkappe an der Außenseite der Bauchdecke 12 gehalten wird. In der Außenseite der Abdeckkappe 36 sind vorzugsweise vom Loch 37 ausgehende rillenförmige Vertiefungen 38 angeordnet, die schlangenförmig verlaufen und insgesamt so lang bemessen sind, daß das gesamte Ende 34 des Schlauches 6 darin eingelegt, d.h.

versenkt werden kann (vgl. Fig. 6).

Der Schlauch 6 und vorzugsweise auch die Abdeckkappe 36 bestehen aus einem gewebeverträglichen Kunststoff, wobei der Konus 32 am Schlauch 6 einstückig angeformt ist. Die Zugglieder 17,18 bestehen aus einem Draht aus korrisionsbeständigem Metall. Es ist auch möglich, die Schlaufe 22 aus einem Kunststoffdraht bzw. -band zu fertigen, weil sich auch ein solcher Werkstoff eignet. Dagegen ist für das Zugglied 17 vorzugsweise ein zwar biegsamer, jedoch eine gewisse Biegesteifigkeit aufweisender Metalldraht vorteilhaft, damit das innere Ende 21 einer Aufbiegung eine Widerstandskraft entgegensetzt.

Die Verpackung 4 kann vor dem Einführen des Schlauches 6 in den Verdauungstrakt von Hand oder im Magen mittels des Gastroskops entfernt werden.

## Patentansprüche

1. Vorrichtung zum Plazieren eines Ernährungs-Schlauches (6) am Magen (16) menschlichen oder tierischen Körpers, bestehend aus
   einer von einer Hülse (8) umgebenen Hohlnadel (9) zum Anbringen einer Öffnung (14) in der Bauchdecke (12) sowie in der Magenwand (13) und zum Einbringen der Hülse (8) in die Öffnung (14),
   dem Schlauch (6), der an seinem einen Ende ein erstes Zugglied (17) aufweist,
   und einem durch die Hülse (8) einführbaren, fadenförmigen zweiten Zugglied (18) zum Einziehen des Schlauches (6) vom Hohlraum (15) des Magens (16) her in die Öffnung (14),
   dadurch gekennzeichnet,
   daß das zweite Zugglied (18) an seinem inneren Ende eine spreizbare Schlaufe (22) und das erste Zugglied (17) ein von der Schlaufe (22) ergreifbares und durch Zusammenziehen der Schlaufe (22) festhaltbares Kupplungselement (21) aufweist.

2. Vorrichtung nach Anspruch 1, dadurch gekennzeichnet, daß wenigstens ein Schenkel (23,24) der Schlaufe (22) unter einer Spannung steht, die ihn vom anderen Schenkel abzubiegen sucht.

3. Vorrichtung nach Anspruch 1, dadurch gekennzeichnet, daß die Schlaufe (22) durch eine Relativverschiebung ihrer Schenkel (23,24) spreizbar ist.

4. Vorrichtung nach einem der Ansprüche 1 bis 3, dadurch gekennzeichnet, daß das dem Schlauch (6) zugeordnete erste Zugglied (17) durch einen Draht, vorzugsweise aus Metall gebildet ist.

5. Vorrichtung nach einem der Ansprüche 1 bis 4, dadurch gekennzeichnet, daß die Länge des ersten Zuggliedes (17) größer bemessen ist als der Abstand zwischen dem Hohlraum (15) des Magens (16) und dem Mund des zu behandelnden Patienten.

6. Vorrichtung nach Anspruch 5, dadurch gekennzeichnet, daß das erste Zugglied im Bereich seines inneren Endes (28) Z-förmig gebogen bzw. gefaltet ist.

7. Vorrichtung nach einem der Ansprüche 1 bis 6, dadurch gekennzeichnet, daß der Schlauch (6), ein gegebenenfalls an seiner Spitze angeordneter Konus (32) und wenigstens teilweise das erste Zugglied (17) einen vorgefertigten Vorrichtungsteil (2) bilden, der insbesondere in einer sterilen, vorzugsweise flexiblen Hülle (4) verpackt ist.

8. Vorrichtung nach einem der Ansprüche 1 bis 7, dadurch gekennzeichnet, daß die Hohlnadel (9) und das zweite Zugglied (18) einen vorgefertigten, vorzugsweise steril verpackten Vorrichtungsteil (3) bilden.

9. Vorrichtung nach einem der Ansprüche 1 bis 8, dadurch gekennzeichnet, daß ihr eine Abdeckkappe (36) zur außenseitigen Abdeckung der Öffnung (14) zugeordnet ist.

10. Vorrichtung nach Anspruch 9, dadurch gekennzeichnet, daß in der Abdeckkappe (36) ein Loch (37) zur Durchführung des Schlauches (6) angeordnet ist.

11. Vorrichtung nach Anspruch 9 oder 10, gekennzeichnet durch eine Klemmvorrichtung zur klemmenden Befestigung des Schlauches (6) an der Abdeckkappe (36).

12. Vorrichtung nach einem der Ansprüche 9 bis 11, dadurch gekennzeichnet, daß die Abdeckkappe (36) außenseitig eine rillenförmige Vertiefung (38) zur Aufnahme des Schlauches (6) aufweist.

13. Vorrichtung nach einem der Ansprüche 9 bis 12, dadurch gekennzeichnet, daß der Schlauch (6) in der Vertiefung (38) einklemmbar ist.

14. Vorrichtung nach Anspruch 12 oder 13, dadurch gekennzeichnet, daß das gesamte von der Abdeckkappe (36) vorstehende Ende (34) des Schlauches (6) in der rillenförmigen Vertiefung (38) einsetzbar ist.

15. Vorrichtung nach Anspruch 1, dadurch gekennzeichnet, daß an dem Schlauch (6) ein Flansch (33) angeordnet ist, der bei im Patienten plaziertem Schlauch an der Innenseite der Magenwand (13) anliegt.

16. Vorrichtung nach Anspruch 15, dadurch gekennzeichnet, daß der innere, orale Schlauchteil (35) eine Schlaufe (40) bildet und mit seinem hinteren Ende (41) lösbar an dem Flansch (33) befestigt ist.

## Claims

1. A device for placing a feeding tube (6) in the stomach (16) of a human being or of an animal comprising:
   - a hollow needle (9) surrounded by a sleeve (8) for making an opening (14) in the abdominal wall (12) and in the wall (13) of the stomach and for inserting the sleeve (8) into the opening (14),
   - the tube (6) which has a first traction member (17) at one end, and
   - a thread-like second traction member (18) which can be introduced through the sleeve (8) for drawing the tube (6) from the interior (15) of the stomach (16) into the opening (14),
   characterised in that
   the second traction member (18) has an expandable loop (22) at its inner end and the first traction member (17) has a coupling member (21) that can be grasped by the loop (22) and held by contracting the loop (22).

2. A device according to claim 1, characterised in that at least one limb (23, 24) of the loop (22) is under a tension which bends it away from the other limb.

3. A device according to claim 1, characterised in that the loop (22) can be expanded by relative displacement of its limbs (23, 24).

4. A device according to any one of claims 1 to 3, characterised in that the first traction member (17) associated with the loop (6) comprises a wire, preferably of metal.

5. A device according to any one of claims 1 to 4, characterised in that the length of the first traction member (17) is greater than the distance between the interior (15) of the stomach (16) and the mouth of the patient to be treated.

6. A device according to claim 5, characterised in that the first traction member is bent or folded into a Z-shape in the region of its inner end (28).

7. A device according to any one of claims 1 to 6, characterised in that the loop (6), a cone (32) that may be arranged at its tip and at least part of the first traction member (17) form a prefabricated part (2) of the device that is sealed in particular in a sterile, preferably flexible cover (4).

8. A device according to any one of claims 1 to 7, characterised in that the hollow needle (9) and the second traction member (18) form a prefabricated, preferably sterile-wrapped part (3) of the device.

9. A device according to any one of claims 1 to 8, characterised in that a cover flap (36) for covering the opening (14) from the outside is associated therewith.

10. A device according to claim 9, characterised in that there is a hole (37) in the cover flap (36) for introducing the tube (6).

11. A device according to claim 9 or claim 10, characterised by a clamping device for clamping fast the tube (6) to the cover flap (36).

12. A device according to any one of claims 9 to 11, characterised in that the cover flap (36) has a groove-shaped depression (38) on the outside for accommodating the tube (6).

13. A device according to any one of claims 9 to 12, characterised in that the tube (6) can be clamped in the depression (38).

14. A device according to claim 12 or claim 13, characterised in that the entire end (34) of the tube (6) projecting from the cover flap (36) can be inserted into the groove-shaped depression (38).

15. A device according to claim 1, characterised in that a flange (33) is arranged on the tube (6) which lies against the inside wall (13) of the stomach when the tube is placed inside the patient.

16. A device according to claim 15, characterised in that the inner, oral part (35) of the tube forms a loop (40) and is mounted detachably with its rear end (41) on the flange (33).

## Revendications

1. Dispositif pour la mise en place d'une sonde alimentaire (6) dans l'estomac (16) du corps humain ou animal, se composant :
   - d'une aiguille creuse (9) entourée d'une gaine (8) pour créer une ouverture (14) dans la paroi abdominale (12) ainsi que dans la paroi (13) de l'estomac et pour introduire la gaine (8) dans l'ouverture (14),
   - de la sonde (6) qui présente sur une de ses extrémités un premier organe de traction (17),
   - et d'un deuxième organe de traction (18) filiforme, pouvant être introduit à travers la gaine (8) pour tirer la sonde (6) hors de la cavité (15) de l'estomac (16) à travers l'ouverture (14),

   caractérisé en ce que le deuxième organe de traction (18) présente sur son extrémité intérieure une boucle expansible (22) et en ce que le premier organe de traction (17) présente un élément de couplage (21) pouvant être saisi par la boucle (22) et pouvant être attaché par resserrement de la boucle (22).

2. Dispositif selon la revendication 1, caractérisé en ce qu'au moins une branche (23, 24) de la boucle (22) se trouve sous une tension tendant à l'écarter de l'autre branche.

3. Dispositif selon la revendication 1, caractérisé en ce que la boucle (22) est expansible au moyen d'un décalage relatif de ses branches (23, 24).

4. Dispositif selon une des revendications 1 à 3, caractérisé en ce que le premier organe de traction (17) associé à la sonde (6) est formé d'un fil, de préférence en métal.

5. Dispositif selon une des revendications 1 à 4, caractérisé en ce que la longueur du premier organe de traction (17) est plus grande que la distance entre la cavité (15) de l'estomac (16) et la bouche du patient à traiter.

6. Dispositif selon la revendication 5, caractérisé en ce que le premier organe de traction est coudé ou replié en forme de Z dans la région de son extrémité intérieure (28).

7. Dispositif selon une des revendications 1 à 6, caractérisé en ce que la sonde (6) un cône (32) disposé, le cas échéant, sur sa pointe, et au moins partiellement le premier organe de traction (17) constituent une partie (2) préassemblée du dispositif, qui est en particulier emballée dans une enveloppe stérile (4) de préférence flexible.

8. Dispositif selon une des revendications 1 à 7, caractérisé en ce que l'aiguille creuse (9) et le deuxième organe de traction (18) constituent une partie (3) préassemblée du dispositif, de préférence sous emballage stérile.

9. Dispositif selon une des revendications 1 à 8, caractérisé en ce qu'il lui est associée une calotte de protection (36) pour recouvrir extérieurement l'ouverture (14).

10. Dispositif selon la revendication 9, caractérisé en ce qu'il est ménagé dans la calotte de protection (36) un trou (37) pour le passage de la sonde (6).

11. Dispositif selon la revendication 9 ou 10, caractérisé par un dispositif de serrage pour fixer la sonde (6) par serrage dans la calotte de protection (36).

12. Dispositif selon une des revendications 9 à 11, caractérisé en ce que la calotte de protection (36) présente sur sa face extérieure un renfoncement (38) en forme de rainure pour recevoir la sonde (6).

13. Dispositif selon une des revendications 9 à 12, caractérisé en ce que la sonde (6) peut être pincée en place dans le renfoncement (38).

14. Dispositif selon la revendication 12 ou 13, caractérisé en ce que toute l'extrémité (34) de la sonde (6) dépassant hors de la calotte de protection (36) peut être insérée dans le renfoncement (38) en forme de rainure.

15. Dispositif selon la revendication 1, caractérisé en ce qu'il est disposé sur la sonde une collerette (33) qui prend appui sur la face interne de la paroi (13) de l'estomac, quand la sonde a été mise en place dans le corps du patient.

16. Dispositif selon la revendication 15, caractérisé en ce que la partie interne, orale (35) de la sonde forme une boucle (40) et est attachée de manière amovible par son extrémité postérieure (41) à la collerette (33).

FIG.1

FIG.2

FIG.3

FIG.4

FIG.5

FIG.6

Fig 7

Fig. 8